# EUROPEAN PATENT SPECIFICATION

(11) **EP 2 959 007 B1**
(45) Date of publication and mention of the grant of the patent: **02.05.2018**
(21) Application number: 14717475.9
(22) Date of filing: 25.02.2014
(51) Int. Cl.: C12Q 1/04, C12M 1/16, C12Q 1/24

(54) **METHOD AND DEVICE FOR DETERMINING MICROBIAL POLLUTION LEVEL IN DIFFERENT ENVIRONMENTS AND PROCESSES**
VERFAHREN UND VORRICHTUNG ZUR BESTIMMUNG DES MIKROBIELLEN SCHADSTOFFGEHALTS IN VERSCHIEDENEN UMGEBUNGEN UND PROZESSEN
PROCÉDÉ ET DISPOSITIF POUR DÉTERMINER UN NIVEAU DE POLLUTION MICROBIENNE DANS DIFFÉRENTS ENVIRONNEMENTS ET PROCESSUS

(43) Date of publication of application: 30.12.2015
(73) Proprietor: Mirheidari, Saeed, Zaeferanieh Tehran (IR)
(72) Inventor: Mirheidari, Saeed, Zaeferanieh Tehran (IR)
(74) Representative: Vernout, Robert
(86) International application number: PCT/IB2014/059225
(87) International publication number: WO 2014/128675

(56) References cited:
- WO-A1-90/02169
- WO-A1-2007/028649
- US-A1- 2009 305 336
- US-B1- 6 627 413
- Oxoid Limited: "Oxoid Dip Slide", Thermo Fischer Scientific Inc. , XP002725554, Retrieved from the Internet: URL:http://www.oxoid.com/UK/blue/prod_deta il/prod_detail.asp?pr=DS0147 [retrieved on 2014-06-10]

## Description

The invention relates to a device for detecting aerobic bacteria, anaerobic bacteria and fungi. Examples of such devices are described in US 2009/305336 A1, WO 2007/028649, US 6 627 413 B1, WO 90/02169 A1, and in the article Oxoid Limited: "Oxoid Dip Slide", Thermo Fischer Scientific Inc. (retrieved from the Internet on 2014-06-10; URL: http://www.oxoid.com/UK/blue/prod_detail/prod_detail.asp?pr=DS0147).

### Background

Microbial growth is a problem in many different places such as hospitals, slaughter houses, chicken farms, dairy farms and factories. Microbes can contaminate solid waste, water, fuel, petrochemical products and industrial fluids. Microbial presence and their metabolic by-products can result in health problems and change in the physical and chemical properties of their host environment. These biomaterials in some systems block the fluid transfer pipes and furthermore can cause chemical deterioration in such systems which results in poor performance and may require costly maintenance. An example is metal-working fluid emulsions or coolants in metal working machines where high microbial growth will cause instability, corrosion, odour and sludge formation resulting in undesirable performance by the fluid. The microbes growing in metalworking fluids could be aerobic bacteria such as Pseudomonas, Bacillus and Staphylococcus, Anaerobic Bacteria such as Desulfovibrio and fungus such as Penicillium spp. and Candid.

Monitoring the microbial load in fluids will help in evaluation of the quality and efficiency of biocides used or the bio-stability of the materials in the fluid. There are different methods to determine the measure of microbial load such as plate count, direct microscopic count, ATP measurement and enzyme activity, which all are common methods in microbiology but are both time consuming and costly. The aforesaid methods will usually require a microbiologist or a person familiar with microbiological methods and also a laboratory setting. In many situations, having a user friendly device for microbial evaluation in a short period of time on site and without having any knowledge of microbiology would be a great advantage.

### Summary of invention

The present invention is a method and a design of a vial for determination of microbial pollution level in different environments and processes. This vial consists of 3 sections for simultaneous detection and evaluation of aerobic bacteria, fungi (yeast and mold) and anaerobic bacteria (sulphate reducing bacteria) growth. In the present invention, aerobic bacteria and fungi population count is based on colony forming on the sides of a slide and comparing them with their presented provided chart. Estimation of the level of anaerobic bacteria growth and population is based on a circular formation at the bottom of the vial which is also compared with a given provided chart. The microbial counts are reported as CFU/ml (Colony Forming Unit per Milliliter).

The present invention provides a suitable device to conduct easy and quick microbial tests in various environments and processes. The device comprises of a plurality sided slide, preferably 2 sided, and a vial. A first side of said plurality sided slide is coated with a first predetermined medium for aerobic bacteria growth and a second side of said plurality sided slide is coated with a second predetermined medium for fungal (mold and yeast) growth. In special cases, a third and more sides of the plural sided slide could be coated with other medium for determination of various aerobic bacteria and fungi. At the bottom of said vial a third predetermined medium is embedded to determine a level of presence of anaerobic bacteria. The slide is inserted into said vial and said slide further comprises a sealing cap to seal the vial to prevent any contamination before and after its use. The media for microbial growth on the slide are prepared under sterile condition and its coated surfaces are free of any contaminant. This tool can be used to test solid surfaces, semisolid materials and fluids based and water containing materials. The different parts of the device are described in the following sections.

### 1- Vial

In figure (1), a 3D image of such a vial is shown. For easy observation, the vial is made of a transparent material such as glass, laminated gypsum or Plexiglas (acrylic glass). At the bottom of the vial there is a single reference point indicating the center of a circular area, wherein said area is divided into plurality of subareas and based on their distance from the said single reference point, each of said subareas represents a predefined level of growth. There are areas of various diameters marked at the bottom of the vial for estimating the level of anaerobic bacteria growth by comparing it with a given provided chart which is provided with the unit. These circles are aimed at estimating the number of anaerobic bacteria. The presence and the level of anaerobic bacteria growth is indicated by a change in the color of the medium in a circular manner and the radius of the circle formed respectively. The cap or the upper section of the device is designed in a manner that it would keep the inside of the vial completely sealed and can have a pop up or screw mechanism.

Furthermore, the slide includes a sharp pointed means such as a needle which is used to carry and deliver samples of a target material into anaerobic medium.

### 2- Slide

In figure (2), the 3D image of such a slide is shown. This slide consists of four main parts.
✔ A side with medium for aerobic bacteria growth
✔ A side with Medium for Fungal (yeast and mold) growth
✔ A needle for inoculating the anaerobic bacteria
✔ The vial cap

On the surface of said plurality sided slide there are build-ups to hold and prevent the medium from cracking or deformation. The medium coated on the surface of the sides of the slide could be coloured so that the colonies would be more noticeable. For example, PCA (plate count agar) is used as a medium for the aerobic bacteria growth. This medium is made of certain materials and a particular pH and has been optimized to allow growth of all types of bacteria and prevent the growth of any types of fungus. A predetermined amount of Cyclohexamide is added to the medium which will help in complete prevention of any fungal growth. Also a predetermined amount of 2, 3, 5-triphenyltetrazollium (TTC) can be added to the medium which will make the colour of the gram positive bacteria colonies to appear as red, so they can be seen more clearly. YM (yeast malt extract medium) which is a special medium for fungal growth is used for the fungal growth evaluation side of the slide. The components and the pH of this medium are selected and optimized in a way that it will allow the complete growth of all types of fungus such as molds and yeasts and will prevent any bacteria growth. To hinder bacteria growth and allow better growth of fungi in this medium, certain concentrations of antibiotics such as chloramphenicol, rose-bengal and streptomycine can be used. The bacterial and fungal growth media of the slide are useful in evaluation of presence of these microbes in industries such as paint, paper, pharmaceuticals, lubricant, and dairy.

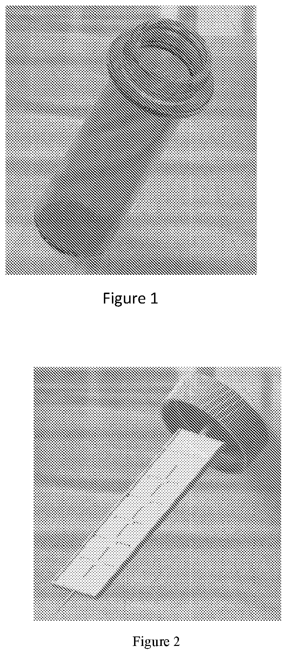

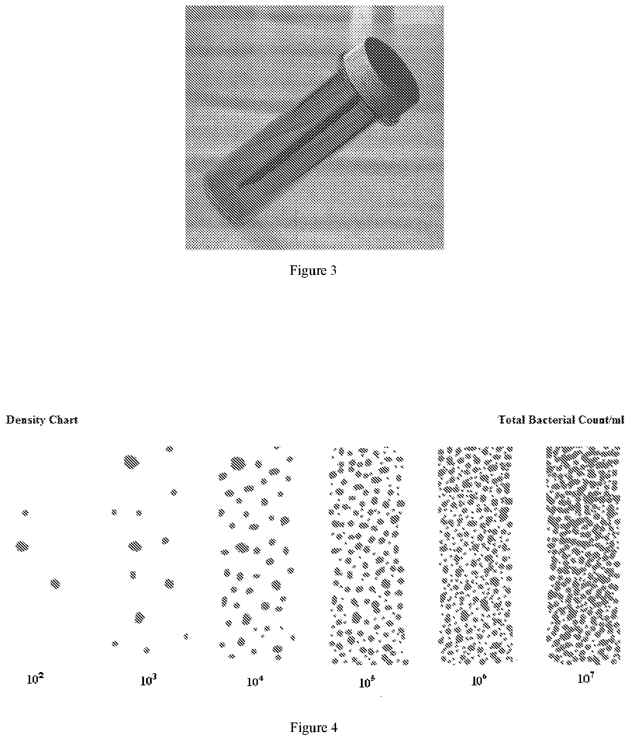

There is a needle placed at the end of the slide. After the slide enters into the test liquid, the liquid will run to the tip of the needle and then the anaerobic medium will get inoculated by the needle entering into it. For better transformation of liquid to the tip of needle, the end of the slide can be made in a triangular or semicircular shape. The complete picture of the device including the slide and transparent vial is shown in fig. 3.

### 3- Anaerobic section of the vial

The anaerobic end which is located at the bottom of the vial to evaluate the anaerobic bacteria constitutes three parts:
✔ A medium for the growth of anaerobic bacteria
✔ Coating layer
✔ An injecting needle

In this part for various anaerobic bacteria, an optimized culture medium for the growth of the specific bacteria may be used. Therefore, they may be used in various industries such as food industries, dairies, pharmacy, paper, metalworking, cooling towers, paint, etc. For example, for anaerobic sulfate reducing bacteria (SRB), which can grow in paper industries, cooling towers and lubricants, leading to spoilage in the system, a TSI medium optimized in the components and pH is used. The TSI medium optimized for the SRB bacteria evaluation turn their color to black as the anaerobic bacteria grow. SRB bacteria convert the sulfate to sulfide in the absence of oxygen. The most common types of these microorganisms are *Desulfovibrio* and *Desulfotormaculum.* The sulfide freed by the bacteria reacts with the iron existing in the anaerobic medium, Forming iron sulfide, which is black in color.

After injection, the anaerobic bacteria grow in the medium in a circular form, and the number of anaerobic microbes in the medium is estimated on the basis of the growth radius and the provided chart provided. The depth of anaerobic bacteria growth medium is 1 to 20 mm thick, preferably 5 to 15 mm, and more preferably 8 to 12 mm).

Among other advantages of the present invention, is counting anaerobic, aerobic microbes and fungi with a single procedure. All the kits available in the market for counting anaerobic bacteria need to be reviewed on a daily basis, but the present invention configured to measure anaerobic, aerobic microbes and fungi within a predetermined time, for example in 48 hours.

A coating layer is used on the anaerobic medium. The layer prevents oxygen from entering the anaerobic part and leads to the creation of a completely anaerobic environment. The coating layer could comprise of paraffin wax or flexible polymers, etc. The coating layer is about 0.1 to 5mm thick and preferably 1 to 2mm thick.

In one embodiment of the present invention, there is a fissure in the center of the (coating layer) at the bottom of the vial where the anaerobic bacteria injection needle is placed. For testing, the slide is taken out of the vial and consequently the needle is taken out of the anaerobic bacteria growth medium, then the slide assembly is impregnated with the targeted sample, and is reinserted in the vial which will result in impregnation of the needle into the anaerobic growth medium at the bottom of the vial and inoculating the same.

Yet in another embodiment of the present invention, the needle attached to the slide is moveable.

In another embodiment of the present invention, the needle can be triggered towards anaerobic the medium by the way of pressing a trigger button arranged on top of the sealing cap without contaminating the needle.

Yet in another embodiment of the present invention, there is not a fissure in the center of the anaerobic part coating layer. Yet in another embodiment of the present invention, the upper portion of the slide is in a bendable form and material.

Yet in another embodiment, slide can have various shapes, for example curved shape, triangular shape so to accommodate a more efficient delivery system for the samples to the tip of the needle.

### Instruction of some examples of applications

**Liquids:** holding the cap of the slide bring out the slide where there is bacterial and fungal growth media on its two sides in a way that there would be no contact with the agar surface. Plunge the slide into the liquid and hold it in contact for 20 to 25 seconds, so that both slides and also its end needle which is for sampling anaerobic bacteria, become thoroughly wet, shake it gently once to remove the excess fluid, return the slide to vial and close it tightly.

**Semisolid:** If the slide is used for emulsions or creams, sampling should be done by a sterile swab. The swab is dipped in the sample and gently rubs on the agar surface. A separate swab should be used for each side.

**Solid area:** Using the bendable slide, holding the slide above the targeted surfaces; pressing it down so the first area of the sides completely touches the surface area of the target; so to extract a sample of the targeted area; and inserting the slide into the vial to continue the test. Follow the same procedure for the other sides of the slide. Touch the targeted area with the needle tip for inoculating the anaerobic growth medium.

The device is put in the incubator after sampling, in a temperature of 30 to 32 degrees centigrade (if an incubator is unavailable, put it in a warm environment with the said temperatures), and the results are checked after 24 hours for bacteria (the time is to be extended until another 24 hours if no growth is observed), after 48 hours for fungi (the time is to be extended until another 24 hours if no growth is observed), and after 48 hours for SRB (the time is to be extended for another 24 hours if the medium does not become black). In case there is not a suitable warm environment and the sample is kept at room temperature (i.e. 25 degrees centigrade), another 24 hours should be added to all the above times.

### Total bacterial count

Nearly all the aerobic bacteria will grow on the side special for aerobic bacteria, which has been made in a way to prevent the fungi growth. In this test, the size of the colonies to be formed is not important, but the number is of interest. If the surface special for bacteria growth is fully covered with bacteria, the sample should be diluted and the test be conducted by the diluted sample, so that we can have a benchmark for the extent of contamination with bacteria in the medium. This generally occurs in liquid mediums. The sample is diluted with a sterile serum (0.9 sodium chloride) by a one to ten proportion (9 milliliter of the sterile serum is used per milliliter of the solution). The medium has been synthesized in a way that most of the gram negative bacteria colonies appear in red; however, if any colony happens to appear in a color other than red it should also be taken into account in counting and estimating the number of bacteria. Note that if only one colony appears it means 10² CFU/ml, and should no colony forms, it means less than 10² CFU/ml. The chart for counting is given in Figure 4.

### Counting molds and yeasts

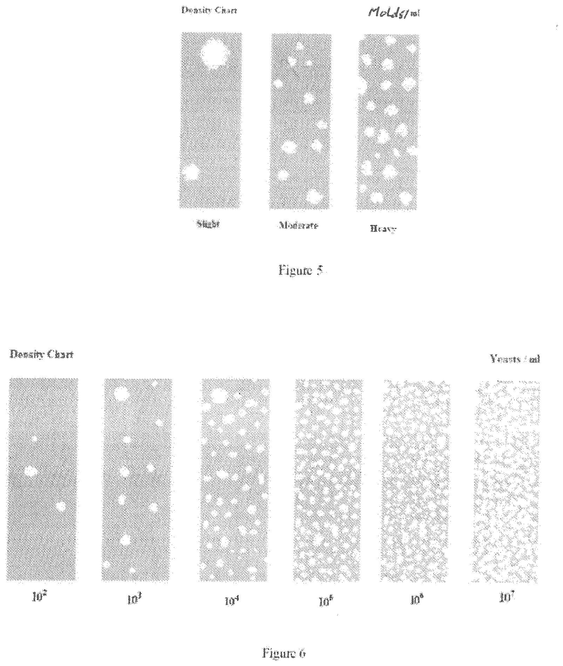

Nearly all types of molds and yeasts grow on the side dedicated for fungi; the side is made so as to prevent the growth of bacteria on the medium. The yeast colonies are generally white, or accompanied by a tint of pink. Determination of the presence of yeasts is similar to bacteria; if even one colony forms, it means 10² CFU/ml, and if no colony form, it will be considered as less than 10² CFU/ml. The chart for estimation is given in Figure 6.
The mold growth is in white, green, brown and black. There is no quantitative measurement of molds. Therefore, its chart has three levels of growth: slight, moderate and heavy. The chart for mold estimation is given in Figure 5.

### Counting anaerobic bacteria

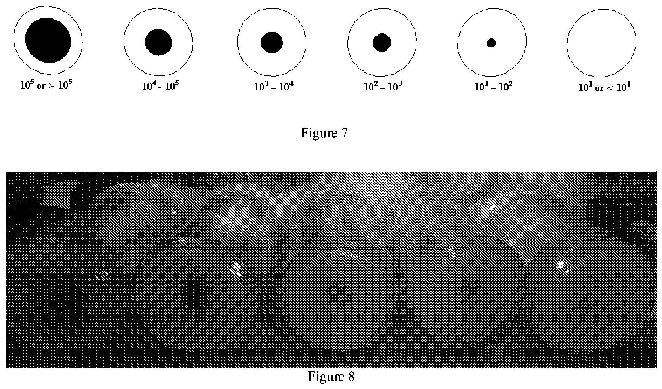

The level of anaerobic bacteria in a contaminated medium is estimated on the basis of the length of the formed circle diameter. As mentioned before, the shift in color for SRB anaerobic bacteria is black. In 48 to 72 hours after the start of the test, we observe the circles formation at the bottom of the vial and compare them with the provided chart to estimate the number of anaerobic bacteria growth. The chart for estimation and counting of SRB anaerobic bacteria is given in Figure 7.

The photo of the lab sample of the anaerobic part is given in Figure 8.

### Advantages

1. This device can be used easily and without need for a trained operator and laboratory equipment;
2. It functions in a reliable and rapid manner
3. Highly safe for the operator
4. Economical to use
5. Designed to be used for liquids, semi-solid and hard surfaces
6. Separate sections for determination of aerobic bacteria, yeasts, molds, and SRB;
7. Very small amounts of sample is needed to perform the test
8. Minimal contact with contaminants outside the sample
9. Easy recognition and estimation of microbes
10. Simultaneous determination of aerobic bacteria, molds, yeasts

## Claims

1. A device for detecting aerobic bacteria, anaerobic bacteria and fungi in a targeted material which comprises of a plurality sided slide, and a vial, wherein a first side of said plurality sided slide is coated with a first predetermined medium for aerobic bacteria growth,
and a second side of said plurality sided slide is coated with a second predetermined medium for fungal (mold and yeast) growth, and wherein at the bottom of said vial a third predetermined medium is embedded to determine a level of presence of anaerobic bacteria; and
wherein the slide comprises a needle, wherein said needle is used to carry and deliver samples of a target material into an anaerobic medium.

2. The device as claimed in claim 1, wherein said slide further comprises a sealing cap to seal the vial to prevent any contamination before and after its use.

3. The device as claimed in claim 1, wherein said vial comprises an opening area for inserting said slide.

4. The device as claimed in claim 1, wherein said slide further comprises a third and more sides which is coated with other mediums for determination of various aerobic bacteria and fungi.

5. The device as claimed in claim 1, wherein said first side is coated with medium for molds and yeasts growth.

6. The device as claimed in claim 1 , wherein said second side is coated with medium for aerobic bacteria growth.

7. The device as claimed in claim 1, wherein said vial contains a special medium for SRB bacteria.

8. The device as claimed in claim 7, wherein medium is deposited at the bottom of the vial, and wherein a predetermined amount of paraffin wax or flexible polymers is deposited on top of said medium to create an anaerobic environment.

9. The device as claimed in claim 8, wherein said device is configured to estimate the number of anaerobic bacteria by detecting changes of attributes comprising colors at the bottom of the vial.

10. The device as claimed in claim 1, wherein said device is configured to estimate the level of yeasts by comparing a number of colonies grown on the second side for yeasts with a first chart provided for yeasts.

11. The device as claimed in claim 1, wherein said device is configured to estimate the level of mold by comparing a number of colonies grown on the second side with a second chart provided for mold.

12. The device as claimed in claim 1, wherein said device is configured to estimate the level of aerobic bacteria by comparing a number of colonies grown on the first side with a third chart provided for aerobic bacteria.

13. The device as claimed in claim 1, wherein said plurality sided slide comprises build-ups on the surface of the slide to prevent medium from detaching and cracking.

## Patentansprüche

1. Eine Vorrichtung zur Detektion aerober Bakterien, anaerober Bakterien und Pilzen in einem Zielmaterial, welche einen mehrseitigen Objektträger und ein Gefäß umfasst, wobei eine erste Seite des mehrseitigen Objektträgers mit einem ersten vorbestimmten Medium für aerobes Bakterienwachstum beschichtet ist und eine zweite Seite des mehrseitigen Objektträgers mit einem zweiten vorbestimmten Medium für Pilzwachstum (Schimmel und Hefe) beschichtet ist und welche am unteren Ende des Gefäßes ein drittes vorbestimmtes Medium eingebettet ist, um ein Maß der Anwesenheit von anaeroben Bakterien zu bestimmen; und
wobei der Objektträger eine Nadel umfasst, wobei diese Nadel zum Übertragen und Einbringen von Proben eines Zielmaterials in ein anaerobes Medium verwendet wird.

2. Die Vorrichtung wie in Anspruch 1 beansprucht, wobei der Objektträger weiterhin eine Verschlusskappe zum Verschluss des Gefäßes umfasst, um jede Kontamination vor und nach seiner Verwendung zu verhindern.

3. Die Vorrichtung wie in Anspruch 1 beansprucht, wobei das Gefäß einen Öffnungsbereich zur Einführung es Objektträgers umfasst.

4. Die Vorrichtung wie in Anspruch 1 beansprucht, wobei der Objektträger weiterhin eine dritte und mehr Seiten, welche mit anderen Medien zur Bestimmung von verschiedenen aeroben Bakterien und Pilzen beschichtet ist, umfasst.

5. Die Vorrichtung wie in Anspruch 1 beansprucht, wobei die erste Fläche mit einem Medium für Schimmel- und Hefewachstum beschichtet ist.

6. Die Vorrichtung wie in Anspruch 1 beansprucht, wobei die zweite Seite mit Medium für aerobes Bakterienwachstum beschichtet ist.

7. Die Vorrichtung wie in Anspruch 1 beansprucht, wobei das Gefäß ein spezielles Medium für SRB-Bakterien enthält.

8. Die Vorrichtung wie in Anspruch 7 beansprucht, wobei Medium am unteren Ende des Gefäßes deponiert ist und wobei eine vorbestimmte Menge von Paraffinwachs oder flexiblem Polymer oben auf dem Medium deponiert ist, um eine anaerobe Umgebung zu schaffen.

9. Die Vorrichtung wie in Anspruch 8 beansprucht, wobei die Vorrichtung zur Abschätzung der Anzahl von anaeroben Bakterien durch Bestimmung von Änderungen von Merkmalen umfassend Farben im unteren Teil des Gefäßes ausgeführt ist.

10. Die Vorrichtung wie in Anspruch 1 beansprucht, wobei die Vorrichtung zur Abschätzung von Hefemenge durch Vergleich einer Anzahl von auf der zweiten Seite für Hefe gewachsenen Kolonien mit einer ersten für Hefen bereitgestellten Abbildung ausgestaltet ist.

11. Die Vorrichtung wie in Anspruch 1 beansprucht, wobei die Vorrichtung zur Abschätzung einer Menge von Schimmel durch Vergleich einer Anzahl von auf der zweiten Seite gewachsenen Kolonien mit einer zweiten für Schimmel bereitgestellten Abbildung ausgestaltet ist.

12. Die Vorrichtung wie in Anspruch 1 beansprucht, wobei die Vorrichtung zur Abschätzung der Menge von anaeroben Bakterien durch Vergleich einer Anzahl von auf der ersten Seite gewachsenen Kolonien mit einer dritten für aerobe Bakterien bereitgestellten Abbildung ausgestaltet ist.

13. Die Vorrichtung wie in Anspruch 1 beansprucht, wobei der mehrseitige Objektträger Aufbauten auf der Oberfläche des Objektträgers zur Verhinderung von Ablösung oder Rissbildung von Medium umfasst.

## Revendications

1. Dispositif pour détecter des bactéries aérobies, des bactéries anaérobies et des champignons dans un matériau ciblé, qui se compose d'une lame à côtés multiples et d'un flacon, dans lequel un premier côté de ladite lame à côtés multiples est enduit d'un premier milieu prédéterminé destiné au développement de bactéries aérobies,
et un deuxième côté de ladite lame à côtés multiples est enduit d'un deuxième milieu prédéterminé destiné au développement de champignons (moisissure et levure), et dans lequel au fond dudit flacon est incrusté un troisième milieu prédéterminé pour déterminer un niveau de présence de bactéries anaérobies ; et
dans lequel la lame comprend une aiguille, dans lequel ladite aiguille est utilisée pour transporter et délivrer des échantillons d'un matériau cible dans un milieu anaérobie.

2. Dispositif selon la revendication 1, dans lequel ladite lame comprend en outre une capsule de fermeture pour fermer hermétiquement le flacon afin d'empêcher toute contamination avant et après son utilisation.

3. Dispositif selon la revendication 1, dans lequel ledit flacon comprend une zone d'ouverture pour introduire ladite lame.

4. Dispositif selon la revendication 1, dans lequel ladite lame comprend en outre un troisième côté et des côtés supplémentaires qui sont enduits d'autres milieux pour la détermination de la présence de diverses bactéries aérobies et de divers champignons.

5. Dispositif selon la revendication 1, dans lequel ledit premier côté est enduit d'un milieu pour le développement de moisissures et de levures.

6. Dispositif selon la revendication 1, dans lequel ledit deuxième côté est enduit d'un milieu pour le développement de bactéries aérobies.

7. Dispositif selon la revendication 1, dans lequel ledit flacon contient un milieu spécial pour les bactéries réductrices de sulfates.

8. Dispositif selon la revendication 7, dans lequel un milieu est déposé au fond du flacon, et dans lequel une quantité prédéterminée de cire de paraffine ou de polymères souples est déposée sur ledit milieu pour créer un milieu anaérobie.

9. Dispositif selon la revendication 8, dans lequel ledit dispositif est configuré pour évaluer le nombre de bactéries anaérobies en détectant des modifications d'attributs comprenant des couleurs au fond du flacon.

10. Dispositif selon la revendication 1, dans lequel ledit dispositif est configuré pour évaluer le niveau de levures en comparant un nombre de colonies développées sur le deuxième côté destiné aux levures avec un premier graphique établi pour les levures.

11. Dispositif selon la revendication 1, dans lequel ledit dispositif est configuré pour évaluer le niveau de moisissure en comparant un nombre de colonies développées sur le deuxième côté avec un deuxième graphique établi pour la moisissure.

12. Dispositif selon la revendication 1, dans lequel ledit dispositif est configuré pour évaluer le niveau de bactéries aérobies en comparant un nombre de colonies développées sur le premier côté avec un troisième graphique établi pour les bactéries aérobies.

13. Dispositif selon la revendication 1, dans lequel ladite lame à côtés multiples comprend des concrétions à la surface de la lame pour empêcher le milieu de se détacher et de se fendiller.
